# EUROPEAN PATENT SPECIFICATION

(11) **EP 2 023 859 B1**
(45) Date of publication and mention of the grant of the patent: **26.12.2012**
(21) Application number: 07756133.0
(22) Date of filing: 27.04.2007
(51) Int. Cl.: A61F 2/24

(54) **Apparatus for cardiac valve replacement**
Vorrichtung für den Ersatz einer Herzklappe
Appareil pour remplacer une valve cardiaque

(30) Priority: 28.04.2006 US 795802 P
(43) Date of publication of application: 18.02.2009
(73) Proprietor: Medtronic, Inc., Minneapolis, MN 55432-5604 (US)
(72) Inventor: RYAN, Timothy, R., Shorewood, Minnesota 55331 (US)
(74) Representative: Hughes, Andrea Michelle
(86) International application number: PCT/US2007/010348
(87) International publication number: WO 2007/127433

(56) References cited:
- US-A- 5 980 570
- US-A1- 2005 273 160
- US-A1- 2006 025 855

## Description

### Technical Field

This invention relates generally to treatment of cardiac valve disease and more particularly to replacement of malfunctioning heart valves.

### Background

Recently, there has been interest in minimally invasive and percutaneous replacement of cardiac valves. In the specific context of pulmonary valve replacement, for example, U.S. Patent Application Publication Nos. 2003/0199971 A1 and 2003/0199963 A1, both filed by Tower, et al. describe a valved segment of bovine jugular vein, mounted within an expandable stent, for use as a replacement pulmonary valve. The replacement valve is mounted on a balloon catheter and delivered percutaneously via the vascular system to the location of the failed pulmonary valve and expanded by the balloon to compress the native valve leaflets against the right ventricular outflow tract, thereby anchoring and sealing the replacement valve. As described in the articles: "Percutaneous Insertion of the Pulmonary Valve", Bonhoeffer, et al., Journal of the American College of Cardiology 2002; 39: 1664 - 1669 and "Transcatheter Replacement of a Bovine Valve in Pulmonary Position", Bonhoeffer, et al., Circulation 2000; 102: 813 - 816, the replacement pulmonary valve may be implanted to replace native pulmonary valves or prosthetic pulmonary valves located in valved conduits. Other articles that describe features of percutaneous valve implantation include Louise Coats, et al., "The Potential Impact of Percutaneous Pulmonary Valve Stent Implantation on Right Ventricular Outflow Tract Re-Intervention," European Journal of Cardio-Thoracic Surgery (England), April 2005, pgs. 536-43; Peter C. Block, et al., "Percutaneous Approaches to Valvular Heard Disease," Current Cardiology Reports (United States), March 2005, pgs. 108-13; Georg Lutter, et al., "Percutaneous Valve Replacement: Current State and Future Prospects," Annals of Thoracic Surgery (Netherlands), December 2004, pgs. 2199-206; Younes Boudjemline, et al., "Percutaneous Pulmonary Valve Replacement in a Large Right Ventricular Outflow Tract: An Experimental Study," Journal of the American College of Cardiology (United States), March 17, 2004, pgs. 1082-7; S. Khambadkone, et al., "Percutaneous Implantation of Pulmonary Valves," Expert Review of Cardiovascular Therapy (England), Nov. 2003, pgs. 541-18; Y. Boudjemline, et al., "Percutaneous Valve Insertion: A New Approach," Journal of Thoracic and Cardiovascular Surgery (United States), March 2003, pgs. 741-2; Philipp Bonhoeffer, et al., "Percutaneous Insertion of the Pulmonary Valve," Journal of the American College of Cardiology (United States), May 15, 2002, pgs. 1664-9; Younes Houdjemline, et al., "Steps Toward Percutaneous Aortic Valve Replacement," Circulation (United States), February 12, 2002, pgs. 775-8; P. Bonhoeffer, et al., "Percutaneous Replacement of Pulmonary Valve in a Right-Ventricle to Pulmonary-Artery Prosthetic Conduit with Valve Dysfunction," Lancet (England), October 21, 2000, pgs 1403-5; P. Bonhoeffer, et al., "Transcatheter Implantation of a Bovine Valve in Pulmonary Position: A Lamb Study," Circulation (United States), August 15, 2000, pgs. 813-6; G.O. Yonga et al., "Effect of Percutaneous Balloon Mitral Valvotomy on Pulmonary Venous Flow in Severe Mitral Stenosis," East African Medical Journal (Kenya), January 1999, pgs. 28-30; and G.O. Yonga, et al., "Percutaneous Transluminal Balloon Valvuloplasty for Pulmonary Valve Stenosis: Report on Six Cases," East African Medical Journal (Kenya), April 19994, pgs. 232-5. US 2006/025855 and US 5,980,570 also teach valve replacement apparatus.

While the approach to pulmonary valve replacement described in the above patent applications and articles appears to be a viable treatment, it is not available to all who might benefit from it due to the relatively narrow size range of available valved segments of bovine jugular veins, which are typically available only up to a diameter of about 22 mm. Unfortunately, the most common groups of patients requiring pulmonary valve replacement are adults and children who have previously undergone transannular patch repair of tetralogy of Fallot during infancy, which left them with right ventricular outflow tracts that are larger than 22 mm in diameter. Thus, typical venous segments cannot typically be securely implanted within these patients.

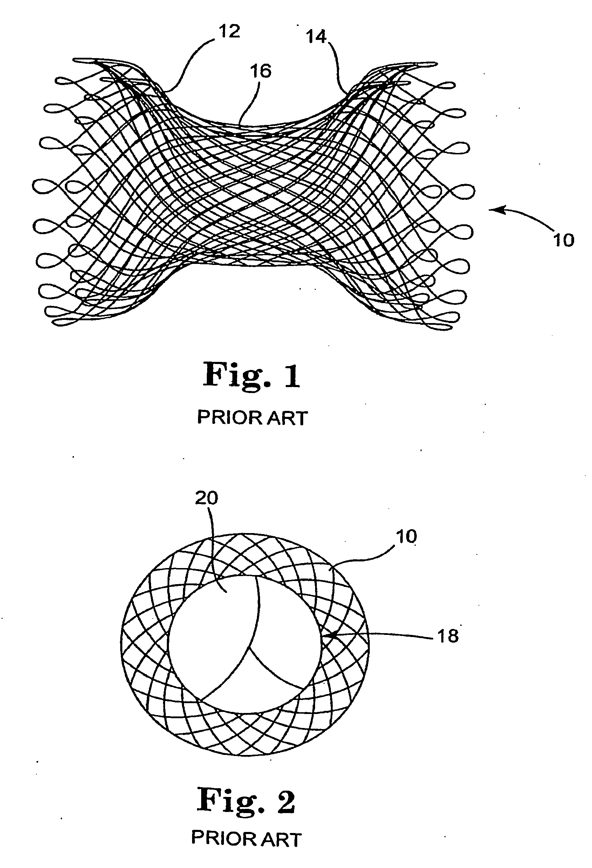

Figure 1 illustrates one example of a prior art adapter stent 10 that has been developed to allow the use of valved segments of bovine jugular veins in a patient with these large right ventricular outflow tracts. The stent 10 comprises a woven wire stent fabricated of nitinol wire, which is heat treated according to conventional techniques to memorize a desired configuration. In the example illustrated, the adapter stent 10 is a generally cylindrical wire structure defining an interior lumen. The adapter stent 10 has generally cylindrical proximal and distal portions 12, 14, each having a diameter that is large enough to contact the inner portion of the outflow tract in which it will be implanted. These proximal and distal portions 12, 14 taper toward a reduced diameter, generally cylindrical central portion 16 in which the valved venous segment or other replacement valve can be mounted.

Figure 2 is an end view of the adapter stent 10 of Figure 1, including a valved venous segment 18 having multiple leaflets 20. The venous segment 18 is sutured to the adapter stent 10 along its proximal and distal edges and may also be sutured to the stent at most, if not all of the intersections of the wire of the stent which overlie the venous segment. Additional sutures have been described as being employed in the areas between the commissures of the valve. One example of an assembly of suitable valve components is described in more detail in Assignee's co-pending U.S. Patent Application titled "Apparatus for Treatment of Cardiac Valves and Method of Its Manufacture", in the names of Philippe Bonhoeffer and Debra Ann Taitague et al., filed November 18, 2005 and published as US2006/206202.

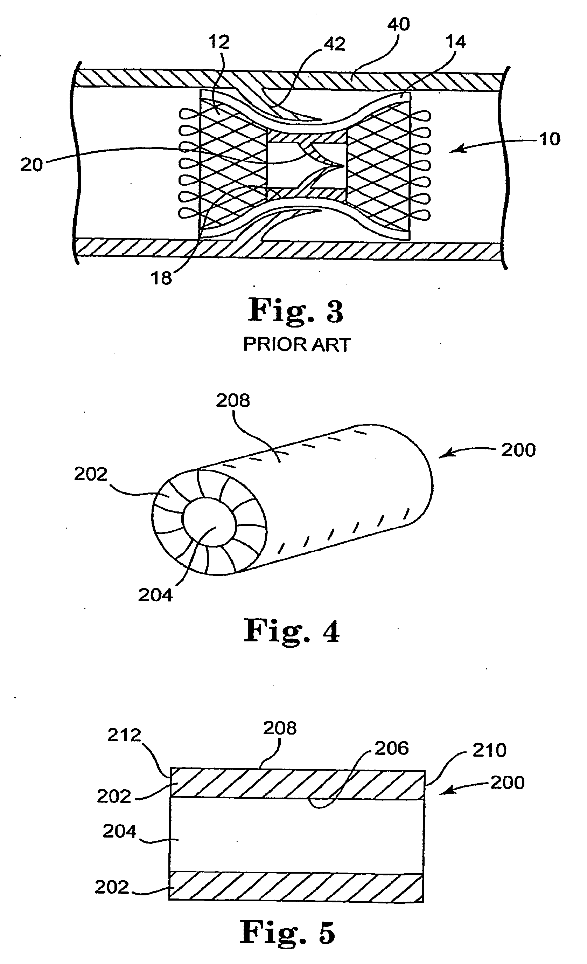

Figure 3 is a schematic cross section of a replacement valve implanted in a right ventricular outflow tract 40, including an adapter stent 10 of the type illustrated in Figure 1. As seen in the Figure, the proximal and distal sections 12, 14 of the adapter stent 10 are positioned so that the larger diameter portions contact the inner wall of the outflow tract 40. The adapter stent 10 can push the native valve leaflets 42 aside, which allows for positioning of the leaflets 20 of the valved venous segment 18 in the original position of the native valve. The adapter stent 10 can also be positioned so that the proximal end segment compresses the native leaflets against the wall of the outflow tract or can also be positioned downstream of the native leaflets 42.

There is, however, a continued need to provide a variety of devices to accommodate the anatomies of different patients, and also a need to improve upon the devices available for implanting valve segments having a desired size and configuration into an area of the patient that has a different size and/or configuration.

### Summary

The present invention is generally intended to provide a mechanism to allow the use of replacement valves in locations in which the size and/or configuration (e.g., diameter, shape, and the like) of the desired location of the replacement valve is different from the size and/or configuration of the available replacement valve. In one particular embodiment, the invention is intended to provide a mechanism that allows the use of valved segments of veins (e.g., bovine jugular veins) as replacement pulmonary valves in patients having large right ventricular outflow tracts. However, the invention may also be useful in conjunction with other replacement valves, such as are disclosed, for example, in U.S. Patent Nos. 6,719,789 and 5,480,424, issued to Cox, or with other valves that comprise pericardial tissue, nitinol, and/or polymers, for other examples. It is further contemplated that segments of porcine or equine veins can be used in conjunction with the devices of the present invention and that mechanical valves can also be used.

The present invention accomplishes the above described objectives by providing an expandable stent as defined in claim 1. Embodiments of the invention are set out in the dependent claims.

In one embodiment, an internal opening is generally cylindrical such that a wall of this internal opening extends along the length of the adapter stent and has an inner diameter that generally corresponds to the outer diameter of a valved venous segment or other replacement valve that is or will be positioned therein.

In one configuration of the invention, a valved venous segment or other replacement valve is positioned within the internal section or opening of an adapter stent prior to implant. In a second configuration, a valved venous segment or other replacement valve is placed in the internal opening of an adapter stent after a previous implant of the adapter stent. In the latter configuration, the replacement valve may itself be mounted in an expandable valve stent, as described in the above cited Tower, et al., applications and Bonhoeffer, et al. articles.

The stents employed in the invention may either be self-expanding stents, such as the type that may be constructed of nitinol or another shape memory material, or may be stents that are expandable by a device such as a balloon. In the embodiments discussed below, an adapter stent of the invention is provided as a tubular structure made of a liquid impermeable outer structure with an internal space for enclosing a substance, such as liquid or gel materials, for an extended period of time. In this way, all blood flow will be directed through the internal section or opening of the adapter stent, where the replacement valve will be positioned.

A method of use of an apparatus for positioning a valve in a tubular organ comprises delivering an expandable tubular adapter to a desired site within the tubular organ, wherein the adapter comprises an enclosed volume surrounded by an outer cylindrical wall having a first diameter that is spaced concentrically from an inner cylindrical wall having a second diameter that is smaller than the first diameter, and first and second end walls extending between the outer and inner cylindrical walls at a proximal and distal end of the adapter, respectively. The method further includes expanding the outer cylindrical walt relative to the inner cylindrical wall so that the outer cylindrical wall contacts the tubular organ, and placing a valve within the inner cylindrical wall of the adapter. The method may further include inserting material into the enclosed volume of the adapter to expand the outer wall relative to the inner wall, which material may include liquid or gel, for example, and may be a material that completely or partially hardens. Alternatively, the valve may be positioned within the inner cylindrical wall of the adapter prior to the adapter being delivered to the desired site.

Also described is an apparatus not forming part of the invention for placing a valve in a tubular organ having a greater diameter than the valve. The apparatus comprises an enclosed volume surrounded by an outer cylindrical wall having a first diameter that is spaced concentrically from an inner cylindrical wall having a second diameter that is smaller than the first diameter, and first and second end walls extending between the outer and inner cylindrical walls at a proximal and distal end of the adapter, respectively. The apparatus further comprises a quantity of material contained within the enclosed volume and a valve mounted within the inner cylindrical wall of the adapter. The inner wall, or both the outer and inner wall include at least one protrusion extending from its surface, such as to mate with at least a portion of the valve.

### Brief Description of the Drawing

The present invention will be further explained with reference to the appended Figures, wherein like structure is referred to by like numerals throughout the several views, and wherein:
Figure 1 is a side view of an exemplary prior art adapter stent;
Figure 2 is a schematic end view of the adapter stent of Figure 1, with a valved venous segment installed therein;
Figure 3 is a cross-sectional side view of a replacement valve including an adapter stent of the type illustrated in Figure 1, as implanted in a right ventricular outflow tract;
Figure 4 is a perspective view of an embodiment of an adapter stent according to the invention;
Figure 5 is a cross-sectional side view of the adapter stent of Figure 4;
Figure 6 is a side view of a stented valved venous segment;
Figure 7 is a side view of a delivery system for a delivering a valved venous segment in accordance with the invention;
Figure 8 is a side view of the valved venous segment of Figure 6 as it can be delivered by the system of Figure 7;
Figure 9 is a cross-sectional side view of a replacement valve positioned within an adapter stent of the invention, as implanted in a right ventricular outflow tract;
Figure 10 is a side view of a delivery system for an adapter stent, according to the present invention;
Figure 11 is a partial cross-sectional view of another embodiment of an adapter stent that includes ribs or protrusions extending outwardly from the outer surface of the adapter stent;
Figure 12 is a partial cross-sectional view of another embodiment of an adapter stent that includes ribs or protrusions extending inwardly toward the inner open channel of the adapter stent;
Figure 13 is a cross-sectional view of an example of an adapter stent according to the invention, including a valve segment positioned therein;
Figure 14 is a schematic perspective view of a valve attached to a stent in accordance with another embodiment of the invention;
Figure 15 is a cross-sectional side view of another embodiment of an adapter stent of the invention, including the valve and stent of Figure 14 in a first position; and
Figure 16 is a cross-sectional side view of the adapter stent of Figure 15, including the valve and stent of Figure 14 in a second position.

### Detailed Description

Referring now to the Figures, wherein the components are labeled with like numerals throughout the several Figures, and initially to Figures 4 and 5, an exemplary configuration of an adapter stent 200 in accordance with the invention is illustrated. Adapter stent 200 can be used to reduce the infundibulum or right ventricular outflow tract to a diameter that accommodates a percutaneous pulmonary valve, for example. That is, adapter stent 200 provides for an area of appropriate size or diameter to accept the implantation of a valve, such as in an area where the infundibulum or right ventricular outflow tract is too large to otherwise accommodate such a valve. Adapter stent 200 includes a generally cylindrical balloon 202 that surrounds an inner channel 204 that extends generally through its center. The inner channel 204, which is defined by an inner wall 206 of balloon 202, is generally concentrically located relative to an outer surface 208 of balloon 202, although it could instead be at least somewhat offset. End walls 210 and 212 extend between the inner wall 206 and outer surface 208, thereby providing an enclosed tubular configuration for balloon 202. End walls 210 and 212 may be generally straight or flat and extend in a generally perpendicular direction from one or both of the inner wall 206 and outer surface 208 toward the other of the inner wall 206 and 208. Alternatively, the end walls 210 and 212 may instead by generally concave or convex portions of the balloon that provide a smooth transition surface between the inner wall 206 and outer surface 208.

Adapter stents of the invention are primarily described herein as being generally tubular in shape for use in pulmonary valve replacement, which will generally involve an adapter have a cylindrical shape with a length for use in the area of a failed pulmonic valve. However, the length and/or shape of the adapter stent can be at least somewhat different when provided for use in replacement of the aortic, mitral or tricuspid valves, all of which are considered to be within the scope of the invention. That is, when used in the mitral valve space, for example, the adapter stent may be much shorter and comprise a more toroid-like shape.

As will be described in further detail below, balloon 202 can be inserted into a patient in a generally deflated or collapsed condition, then subsequently filled with one or more of a variety of substances. For example, these substances may be of a type that does not harden, such as air or liquid of varying viscosities. In these cases, the balloon can be provided with a mechanism to keep the material contained within the balloon (i.e., to prevent leakage), such as a plug or other closing mechanism. It is also contemplated that the balloon itself is made of a self-sealing type of material that can be punctured or otherwise compromised to allow filling of the balloon through a nozzle or other device, and that will reseal itself after removal of the balloon-filling device. Alternatively, the balloon can be filled with a compound that is completely or partially hardenable such that it cannot leak or otherwise migrate from the balloon once it has hardened. Such hardenable materials may harden quickly or instantaneously within the balloon after it is injected or inserted therein, or the materials can gradually harden over time, such as in response to the temperature of the surrounding bodily fluids and tissues. Other exemplary materials that may be used within the balloon include saline, collagen, silicone, hydrogel, blood, foam, beads or spheres made of glass, polymers, or metals, or the like.

Although the balloon and/or adapter stent are described above as being generally cylindrical in shape, it is understood that the balloon may instead be shaped in a number of different ways that are considered to be within the scope of the invention. For example, the balloon may have an outer wall that is generally elliptical, oval, spherical, or irregularly shaped, for example, and the inner wall of such a balloon may have a similar or different shape from the outer wall.

In one specific example, the outer wall of an adapter stent may be generally oval or D-shaped to conform to a patient's generally D-shaped mitral valve opening. Such an adapter can facilitate usage of a circular or other shaped replacement valve. In yet another specific example, a heart failure patient may have a dilated round mitral orifice that can be remodeled back to be more D-shaped or oval with the use of an appropriately shaped adapter stent. This type of remodeling of the shape of a valve opening can also be beneficial for congenital heart valve patients who desire to have the valve anatomy remodeled to accommodate a new replacement valve and/or to improve blood flow, hemo dynamics, and the like.

In accordance with the invention, the inner wall of an adapter stent is configured to accommodate a valve, and the outer wall is configured so that a sufficient portion of its area will securely contact the body opening in which it is inserted. That is, the outer wall of the balloon can have a number of irregularly-shaped contours such as may be necessary to accommodate the congenital irregularities of a right ventricular outflow tract, for example. In that regard, the balloon and/or adapter stent may have an outer wall that appears to be generally cylindrical when in its collapsed or semi-collapsed condition, but that is relatively conformable such that its outer wall will be relatively irregular when expanded within the appropriate body opening. Thus, the adapter stents of the invention may be used in areas of the body that do not comprise regularly or symmetrically shaped tubular openings. Further, with any of these balloons and/or adapter stents, the inner channel may be somewhat or significantly offset (i.e., non-concentric) relative to the outer surface of the balloon.

The balloon 202 can be constructed of any material that is compatible with the material that it contains, and is preferably impermeable or semi-impermeable to bodily fluids. In any of the embodiments of the invention, the balloon can be made of one or more materials that form a continuous tube that can be maintained in its expanded state for an extended period of time. That is, the material placed within the inner area of the balloon preferably does not migrate or leak out once the balloon has been sealed, and the fluids outside.the balloon preferably do not migrate into the inner area of the balloon. In other words, the material from which the balloon is made is preferably impermeable to any of the fluids with which it comes in contact. Exemplary balloon materials include PTFE or ePTFE, although a wide variety of impermeable materials or combinations of materials can be used. It is further contemplated that the surface of the balloon can include a material that facilitates tissue in-growth or pannus, such as a fabric or other material that has a biocompatible and biostable coating and/or surface texture that facilitates healing of the balloon in the location where it was inserted. Such a material may make up the entire balloon, or only a portion of the balloon may include a material that facilitates tissue in-growth.

In one configuration of the invention, the material from which the balloon 202 is constructed is flexible enough to accommodate a wide variety of anatomies so that an adapter stent 200 of one particular size and shape can be configured for use in a wide variety of patients and/or anatomical areas of patients. In addition, the balloon 202 is desirably designed in such a way that it provides an inner channel 204 having a predetermined size when it is inflated, no matter how far the inner wall 206 and outer surface 208 are spaced from each other. That is, if the balloon 202 is to be expanded to accommodate an unusually large anatomy, the inner channel 204 can be maintained at a predetermined diameter to accept a particular valve in its proper orientation. Thus, it is possible that the balloon 202 is constructed of a single material or a combination of materials, parts, and/or features that vary in thickness or other properties in certain areas of the balloon to allow for a desired expansion profile. For example, the portion of the balloon 202 that makes up the inner wall 206 can be relatively non-deformable or non-expandable as compared to the portion of the balloon that makes up the outer surface 208 so that addition of material to the inner area of the balloon 202 will not allow expansion of the balloon 202 into the inner channel 204, but will only allow for expansion of the outer surface 208 of the balloon 202 away from the inner wall 206. In this way, the diameter of the inner channel 204 can be maintained at a particular size and shape for accepting a replacement valve. In addition, it is preferable that the distance between the end wall 210 and the end wall 212 will be approximately the same when the balloon 202 is collapsed or when the balloon 202 is partially or completely expanded. However, it is also possible that the length of the balloon 202 increases at least slightly when material in inserted therein.

The expansion of the balloons of the invention may involve an actual stretching or expansion of the material from which the balloon is made in response to an addition of material into its internal volume. However, in other embodiments, the material itself may not actually expand or stretch, but the filling of the internal volume of the balloon instead causes the walls of the balloon to move away from each other, thereby expanding the internal balloon volume.

The balloon 202 can be covered or partially covered with one or more substances to control or prevent ingrowth and sealing of the valve, such as Dacron, PTFE, tissue, and the like. The material from which the balloons are made may include a material that has essentially zero porosity when first used, but which allows some short-term, limited leakage prior to implantation. This type of material becomes impermeable when implanted. Metal stent material can also be used in combination with the balloon material to allow tailored radial force for the balloon 202.

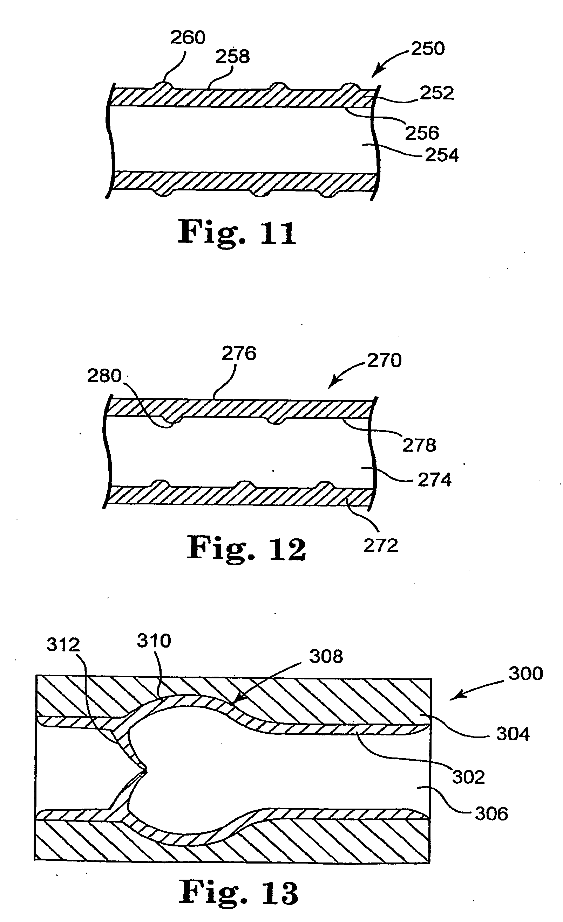

The adapter stents of the invention include features such as rings, barbs, hooks, teeth, or other protrusions or recesses that extend from the balloon material of the inner wall. The adapter stents of the invention can also include features such as rings, barbs, hooks, teeth, or other protrusions or recesses that extend from or into the balloon material of the outer wall. One example of a configuration of the outer wall is illustrated as an adapter stent 250 in Figure 11. Adapter stent 250 includes a generally cylindrical balloon 252 that surrounds an inner channel 254 that extends generally through its center. Balloon 252 includes an inner wall 256 that defines the inner channel 254, where the inner channel 254 has a generally constant diameter along its length. Balloon 252 further includes an outer wall 258 spaced from inner wall 256. At least one protrusion 260 extends outwardly from the outer wall 258, which can be provided as discrete bumps or knobs, for example, or may include ribs that extend around all or some portion of the periphery of the balloon 252. The protrusions 260 can be spaced from each other, as shown, or can be more of a continuous textured surface of the outer wall 258. Another alternative configuration of these protrusions 260 includes one or more spiral ribs that extend continuously or semi-continuously along the length of the balloon 252. Other configurations of these protrusions may also be provided that allow for the performance characteristics described below.

The number, spacing, and particular configurations of any protrusions 260 from outer wall 258 are chosen to provide and/or enhance certain features of an adapter stent relative to a certain procedure. That is, these protrusions can be provided to increase the radial force of the balloon 252, reduce its migration risk, and/or improve the overall structural integrity of the adapter stent, for example. Any protrusions 260 that are provided may be formed integrally with the outer wall 258, or may be adhered or otherwise attached to the balloon 252, using the same or different materials as the material from which the balloon is constructed. One example of such an alternative protrusion is a plug that extends into and from the outer wall 258, such as a self-expandable cylindrical mesh device of the type commercially available from AGA Medical Corporation of Golden Valley, Minnesota, as the "AMPLATZER Vascular Plug".

Figure 12 illustrates a portion of an embodiment of an adapter stent 270, which comprises a balloon 272 surrounding an inner channel 274 that extends generally through its center. Balloon 272 includes an outer wall 276 spaced from an inner wall 278 that defines the inner channel 274. Outer wall 276 has a generally constant diameter along its length; however, inner wall 278 includes at least one protrusion 280 extending into the inner channel 274. Protrusions 280 may include any of the variations described or contemplated above relative to protrusions from outer wall 258 of adapter stent 250, as desired. One function for such protrusions 280 is to control the position of a new valve within the adapter stent, such as to prevent migration of the valve. That is, such protrusions can promote docking, positioning, and/or securing of the valve within the adapter stent. Further, a single adapter stent may use a combination of protrusions from both its inner and outer walls and along all or a portion of these wall lengths.

Figure 13 illustrates another example of an adapter stent 300, which further includes a valve segment 302 positioned therein. Adapter stent 300 includes a balloon 304 surrounding a generally cylindrical inner channel 306. Inner channel 306 includes at least one contoured portion 308, which thereby varies the diameter of the inner channel 306 along a portion of its length. In one embodiment, a discrete contoured portion 308 is provided to correspond with each leaflet of a particular valve that will be used therewith. That is, if a three-leaflet valve will be used, for example, three corresponding contoured or bulbous portions 308 can be provided. The contoured portions 308 can thereby correspond with the anatomic or natural shape of a valve to be inserted therein. However, the contoured portions 308 may instead be more continuous around all or a portion of the inner periphery of the balloon 304. Such contoured portions may alternatively or additionally be provided on the outer wall of an adapter stent to accommodate the anatomy of the patient.

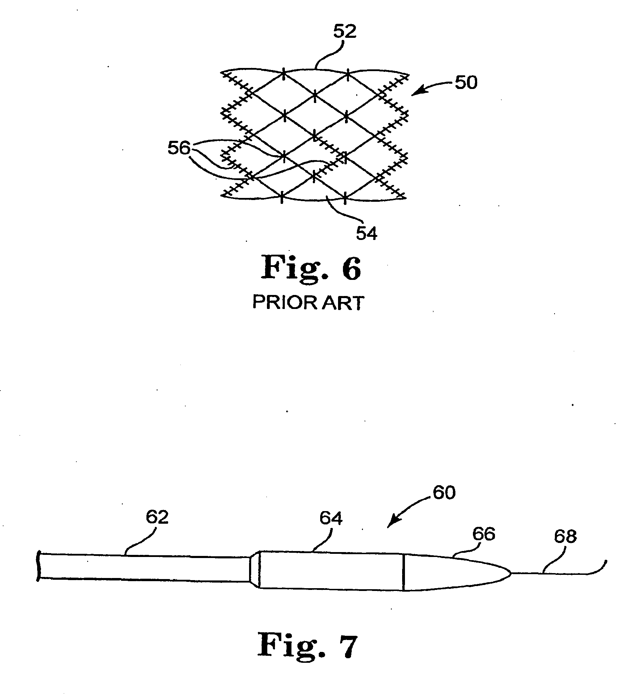

Figure 6 illustrates another example of a stented valve venous segment 50 that has been developed, which can be positioned within a previously implanted adapter stent, such as the adapter stent 200. The stented venous segment 50 may correspond to that described in the above-cited Tower, et al., and Bonhoeffer et al. references, and generally comprises a stent 52 and a venous segment 54. The stented venous segment 50 is expandable to an outer diameter as large as the diameter inner channel 204 of adapter stent 202. The stent 52 may be fabricated of platinum, stainless steel or other biocompatible metal. While it may be fabricated using wire stock as described in the above-cited Tower, et al. applications, it can also be produced by machining the stent from a metal tube or molding the stent from another appropriate material. The venous segment 54 is mounted within the stent 52 with its included valve located between the ends of the stent and is secured to the stent by sutures 56. Sutures 56 are located at the proximal and distal ends of the stent and preferably at all or almost all of the intersections of the stent, as illustrated. A more detailed description of the manufacture of stented venous segments is disclosed in Assignee's co-pending U.S. Patent Application titled "Apparatus for Treatment of Cardiac Valves and Method of Its Manufacture", in the names of Philippe Bonhoeffer and Debra Ann Taitague et al., filed November 18, 2005 and published as US 2006/206202.

Figure 7 illustrates one exemplary system for delivering a valved venous segment of the type shown in Figure 6 to the interior of a previously implanted adapter stent, such as adapter stent 200. The delivery system 60 comprises an outer sheath 62 overlying an inner balloon catheter (not visible in this Figure). The outer sheath includes an expanded distal portion 64, within which the stented valved venous segment is located. The venous segment is compressed around a single or double balloon located on the inner catheter. A tapered tip 66 is mounted to the distal end of the inner catheter and serves to ease the passage of the delivery system through the patient's vasculature. The system also includes a guidewire 68, which can be used to guide the delivery system to its desired implant location.

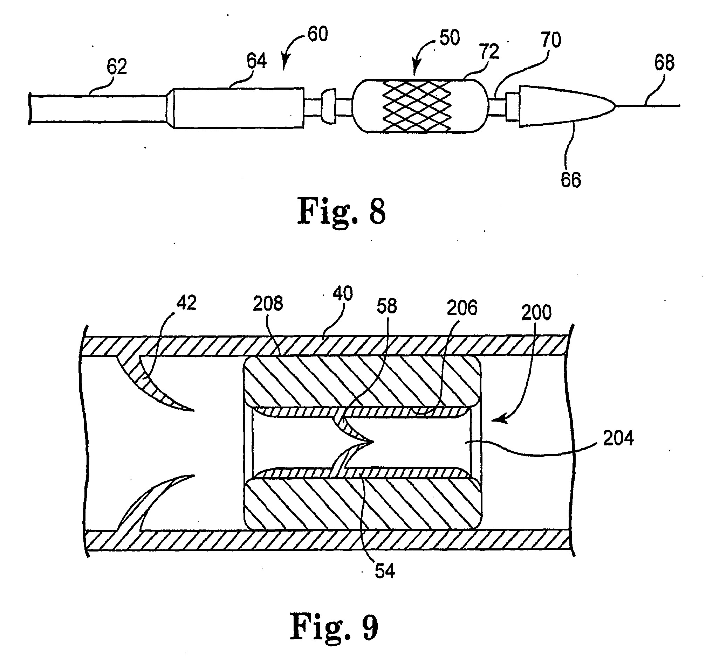

The delivery system of Figure 7 and its use may correspond to that described in the above-cited Tower, et al. applications, with the exception that the venous segment is placed within the middle section of a previously placed adapter stent, such as adapter stent 200, rather than expanded against a failed native or prosthetic valve. The delivery system can be advanced to the desired valve implant site using the guidewire 68, after which the sheath 62 is retracted to allow balloon expansion of the venous segment, as illustrated in Figure 8.

Figure 8 illustrates the mechanism for deployment of a stented valved venous segment, such as segment 50, within middle portion of a previously implanted adapter stent, such as adapter stent 200. The outer sheath 62 is moved proximally, exposing the balloon 72 mounted on inner catheter 70. The balloon 72 is expanded, which thereby expands venous segment 50 against the inner surface of the previously implanted adapter stent, stabilizing and sealing the venous segment within the adapter stent. Since protrusions or other docking features are provided within the adapter stent, the venous segment 50 can be engaged with such features. The balloon is then deflated and the delivery system is withdrawn proximally.

Figure 9 is a schematic cross-sectional view of a replacement valve, as implanted in a right ventricular outflow tract 40 within an adapter stent 200 of the invention. As discussed above, this replacement valve can either be implanted within the adapter stent 200 at the same time the adapter stent is implanted in the patient, or the replacement valve can instead be implanted at some time after the adapter stent 200 is implanted. In yet another alternative, the adapter stent and/or replacement valve can be placed surgically within the patient, with the two components being implanted in a single procedure or multiple procedures. In any case, Figure 9 illustrates the outer surface 208 of the adapter stent 200 expanded against the inner wall of the outflow tract 40. As set out above, the inner channel 204 preferably maintains a particular diameter that is appropriate for holding and maintaining a chosen replacement valve. Thus, depending on the size of the outflow tract 40, the outer surface 208 may be relatively close to the inner channel 204 or may be spaced relatively far from the inner channel 204. In other words, if the outflow tract is relatively large, the outer surface 208 will be spaced relatively far from the inner channel 204 as compared to a configuration where the outflow tract is not as large.

With the adapter stent 200, the outer surface 208 is preferably in contact with the inner surface of the outflow tract 40 along the entire length of the stent, although it is possible that portions of the outer surface 208 are not in contact with the outflow tract. In any case, enough of the outer surface 208 should be in contact with the outflow tract 40 to accomplish sealing and prevent its migration after implantation. The adapter stent in Figure 9 is mounted downstream of the native valve leaflets 42 to allow them to continue to function during the time the adapter stent 200 and the stented venous segment 50 are being implanted. Optionally, the venous segment 50 may be placed within the adapter stent 200 at some period of time after its initial implant, such as several days or weeks. In this Figure, the leaflets 58 of an implanted venous segment 54 are also illustrated within the adapter stent 200.

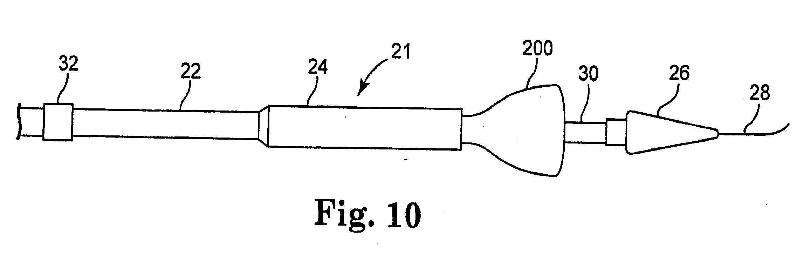

Figure 10 illustrates one exemplary system that may be used for delivering the adapter stents which can be somewhat similar to the system used for delivering a venous segment. The delivery system 21 comprises an outer sheath 22 overlying an inner catheter (not visible in this Figure). The outer sheath 22 has an expanded distal portion 24, within which an adapter stent 200 (with or without a valved venous segment) can be located. The adapter stent 200 can be initially be in its collapsed condition, compressed around the inner catheter, and retained in its compressed configuration by the outer sheath 22. A tapered tip 26 is mounted to the distal end of the inner catheter and serves to ease the passage of the delivery system 20 through the vasculature. The system also includes a guidewire 28, which may be used to guide the delivery system 20 to its desired implant location.

Delivery system 21 further includes a mechanism 32 that communicates with an adapter stent for its inflation or expansion at the desired implant site. The mechanism 32 can include a wide variety of devices that can provide the desired material to the interior of the adapter stent 200, such as a pump that can move fluid or gel into the adapter stent 200, a source of pressurized air or other gas that can be controlled to inflate the adapter stent 200 by a predetermined amount, and the like. That is, the material that is used within the adapter stent 200 will determine the type of mechanism 32 that needs to be used to inflate it or expand it. The delivery system 21 and/or the adapter stent 200 can optionally be provided with a sealing mechanism (not shown) for sealing or closing any openings in the adapter stent 200 after material is injected or inserted therein to keep the material from leaking out of the stent 200.

The outer sheath 22 can be moved proximally, either in response to the expansion of the adapter stent 200 via the mechanism 32, or by pulling it from one end, thereby allowing the adapter stent 200 to expand away from the inner catheter 30, which is visible in this configuration of the device. The distal segment of the adapter stent 200 can engage the wall of the heart vessel at the desired implant site, stabilizing the stent. The outer sheath 22 is then moved further proximally, releasing the proximal segment of the adapter stent, which is then free to expand in diameter until it contacts the wall of the heart vessel. Material can continue to be added to the adapter stent 200 until it is inflated or expanded to its desired size. The delivery system is then withdrawn proximally. In certain configurations, the valved venous segment is pre-mounted within the adapter stent 200, so this inflation or expansion of the adapter stent 200, with its valved venous segment mounted therein, provides a single-procedure implantation of the replacement valve. Alternatively, the valved venous segment can be inserted into the adapter stent in a separate procedure.

The stented valved venous segments used with the adapter stents of the invention have been described and shown as being compressible for installation into a patient, then expandable, such as by a balloon or otherwise expandable portion of a delivery system. However, it is also understood that other types of stented valves can be used, such as those that are referred to as the "self-expanding" type. These self-expanding stents are compressible for installation into a patient, then will radially expand to a desired size simply by removing certain external forces that were used to keep the stent in a compressed state. Other types of stented valves can also be used that are compressible and expandable in ways other than those described herein.

Referring again to Figure 13, the valve segment 302 is illustrated as it has been pre-attached or mounted within the inner channel 306 of the balloon 304 so that the implantation procedure can be accomplished in a single step. That is, rather than first installing the adapter stent, then subsequently installing a valve segment within its inner channel, Figure 13 illustrates a adapter stent assembly that allows the surgeon to eliminate the separate step of using a delivery system with a balloon to expand the valve segment. Valve segment 302 is illustrated in the general form of a bovine jugular vein that includes bulbous areas 310 that can expand into the contoured portions 308 of the balloon 304. This configuration can provide less stress on the leaflets 312 since the bulbous areas 310 are not compressed or otherwise deformed into the inner channel 306 of the balloon 304, but are allowed to remain generally in their native anatomical form.

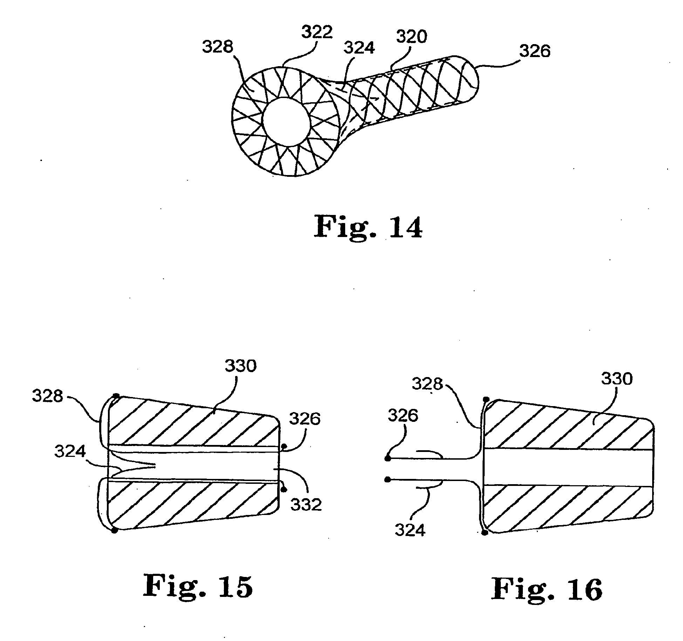

Figure 14 illustrates an embodiment of a valve 320 (shown with broken lines) attached within a stent 322 in accordance with another embodiment of the invention. Valve 320 includes leaflets 324, and the stent includes a proximal end 328 and an opposite distal end 326. The proximal end 328 may be at least partially covered with tissue, for example, such as during a procedure of securing the stent 322 to the valve 320 by a sewing operation. The stent 322 may include a braided or wire mesh material, or any other appropriate stent material. Figure 15 shows this valve-stent assembly of Figure 14 positioned within an adapter stent or balloon 330, with the leaflets 324 positioned generally within an inner channel 332 of the balloon 330, and the proximal end 328 of the stent 322 extending beyond one end of balloon 330. Stent 322 is attached to the balloon 330, such as by sewing the proximal end 328 of stent 322 to balloon 330 around at least a portion of the circumference of the stent 322. The balloon 330 can be provided with a cuff or other extension (not shown) for attachment of a stent without compromising any of the strength of the balloon, for example. At this point, the adapter stent having a stented valve installed therein can be shipped to a clinician, for example.

The valve-stent assembly of Figure 14 can optionally be inverted to the configuration shown in Figure 16 by pulling the distal end 326 of the stent 322 through the inner channel 332 of the balloon 330 until it is essentially turned inside out as compared to Figure 15. In this way, the balloon with an attached stented valve can be compressed to a smaller dimension for delivery of the system into a patient, because a smaller volume of material will be in this area than if the stented valve were to remain in the inner channel 332 during the compression process. The adapter stent may then be used in a similar way as discussed herein relative to other embodiments of the invention, such as will include expanding the balloon with a material, removing any delivery devices, etc., but can also include the step of reversing the inversion process described above by pressing the distal end 326 of the stent 322 back into the inner channel 332 so that it can function as a valve for the patient

Finally, while the invention described above is particularly optimized for placement of valves in the right ventricular outflow tract, it is possible that the invention might be used to place valves in other blood vessels or other tubular organs. Similarly, while bovine jugular veins are disclosed as the source for the valved segments used to practice the invention, other source animals or source vessels may be substituted. Also, polymer or thin metal film valves may be used. Further, alternative exemplary replacement valves can be used, of the type described U.S. Patent Nos. 6,719,789 and 5,480,424, issued to Cox, discussed above. As such, the above description should be taken as exemplary, rather than limiting.

The present invention has now been described with reference to several embodiments thereof.

## Claims

1. An apparatus for positioning a valve in a tubular organ having a greater perimeter than the valve, comprising:
an adaptor comprising an outer cylindrical wall (208) having a first perimeter that is spaced concentrically from an inner cylindrical wall (206) having a second perimeter that is smaller than the first perimeter, and first and second end walls (210, 212) extending between the outer and inner cylindrical walls at a proximal and a distal end of the adapter, respectively, the outer and inner cylindrical walls and the first and second end walls together defining an enclosed volume;
a quantity of material contained within the enclosed volume;
a valve (320) mounted within the inner cylindrical wall of the adapter;
and **characterised by** comprising at least one integrally formed protrusion (260, 280) extending outwardly, with respect to the enclosed volume, from the inner cylindrical wall.

2. The apparatus of claim 1, wherein outer and inner cylindrical walls and the first and second end walls extending between the outer and inner cylindrical walls comprise a liquid resistant material.

3. The apparatus of claim 1, wherein the material contained within the enclosed volume is a liquid material.

4. The apparatus of claim 1, wherein the material contained within the enclosed volume is a gel material.

5. The apparatus of claim 1, wherein the material contained within the enclosed volume is at least semi-solid.

6. The apparatus of claim 1, wherein the outer cylindrical wall comprises at least one protrusion (260) extending from its surface.

7. The apparatus of claim 1, wherein the at least one protrusion extending from the surface of the inner cylindrical wall is configured to mate with at least a portion of the valve.

## Patentansprüche

1. Vorrichtung zum Positionieren einer Klappe in einem röhrenförmigen Organ, das einen größeren Umfang als die Klappe hat, wobei die Vorrichtung Folgendes umfasst:
einen Adapter, der eine äußere zylinderförmige Wand (208) mit einem ersten Umfang, die konzentrisch von einer inneren zylinderförmigen Wand (206) mit einem zweiten Umfang beabstandet ist, der kleiner als der erste Umfang ist, und eine erste und eine zweite Stirnwand (210, 212) umfasst, die sich zwischen der äußeren und der inneren zylinderförmigen Wand an einem proximalen bzw. distalen Ende des Adapters erstrecken, wobei die äußere und die innere Wand und die erste und zweite Stirnwand zusammen ein geschlossenes Volumen abgrenzen;
eine Materialmenge, die in dem geschlossenen Volumen eingegrenzt ist;
eine Klappe (320), die innerhalb der inneren zylinderförmigen Wand des Adapters angebracht ist;
und die **dadurch gekennzeichnet ist, dass** sie mindestens einen angeformten Vorsprung (260, 280) umfasst, der sich bezüglich des geschlossenen Volumens von der inneren zylinderförmigen Wand nach außen erstreckt.

2. Vorrichtung nach Anspruch 1, wobei die äußere und die innere zylinderförmige Wand sowie die erste und die zweite Stirnwand, die sich zwischen der äußeren und der inneren zylinderförmigen Wand erstrecken, aus einem flüssigkeitsbeständigen Material bestehen.

3. Vorrichtung nach Anspruch 1, wobei das Material, das in dem geschlossenen Volumen eingegrenzt ist, ein flüssiges Material ist.

4. Vorrichtung nach Anspruch 1, wobei das Material, das in dem geschlossenen Volumen eingegrenzt ist, ein Gelmaterial ist.

5. Vorrichtung nach Anspruch 1, wobei das Material, das in dem geschlossenen Volumen eingegrenzt ist, mindestens halbfest ist.

6. Vorrichtung nach Anspruch 1, wobei die äußere zylinderförmige Wand mindestens einen Vorsprung (260) umfasst, der sich von ihrer Oberfläche erstreckt.

7. Vorrichtung nach Anspruch 1, wobei der mindestens eine Vorsprung, der sich von der Oberfläche der inneren zylinderförmigen Wand erstreckt, so konfiguriert ist, dass er mit zumindest einem Teil der Klappe zusammengefügt werden kann.

## Revendications

1. Appareil pour positionner une valve dans un organe tubulaire ayant un périmètre supérieur à la valve, comprenant :
un adaptateur comprenant une paroi externe (208) cylindrique ayant un premier périmètre qui est espacé concentriquement de la paroi interne (206) cylindrique ayant un deuxième périmètre qui est inférieur au premier périmètre, et la première et deuxième parois (210, 212) s'étendant entre les parois cylindriques externe et interne à une extrémité proximale et distale de l'adaptateur, respectivement, les parois cylindriques externe et interne et la première et deuxième parois d'extrémité définissant ensemble un volume renfermé ;
une quantité de produit contenu dans le volume renfermé ;
une valve (320) montée dans la paroi cylindrique interne de l'adaptateur ;
et **caractérisé en ce qu'**il comprend au moins une protubérance (260, 280) intégralement formée s'étendant vers l'extérieur, par rapport à un volume renfermé, à partir de la paroi interne cylindrique.

2. Appareil selon la revendication 1, dans lequel les parois cylindriques interne et externe et la première et deuxième parois d'extrémité s'étendant entre les parois cylindriques interne et externe comprennent un produit liquide résistant.

3. Appareil selon la revendication 1, dans lequel le produit contenu dans le volume renfermé est un produit liquide.

4. Appareil selon la revendication 1, dans lequel le produit contenu dans le volume renfermé est un gel.

5. Appareil selon la revendication 1, dans lequel le produit contenu dans le volume renfermé est un produit au moins semi-solide.

6. Appareil selon la revendication 1, dans lequel la paroi externe cylindrique contient au moins une protubérance (260) s'étendant de sa surface.

7. Appareil selon la revendication 1, dans lequel l'au moins une protubérance s'étendant de la surface de la paroi interne cylindrique est configurée pour s'accoupler avec au moins une partie de la valve.
